# EUROPEAN PATENT SPECIFICATION

(11) **EP 0 896 222 B1**
(45) Date of publication and mention of the grant of the patent: **27.12.2006**
(21) Application number: 98202633.8
(22) Date of filing: 04.08.1998
(51) Int. Cl.: G01N 33/04

(54) **System for monitoring and controlling protein utilization in animals**
System zur Überwachung und Steuerung der Eiweissbenutzung bei Tieren
Système de surveillance et contrôle de l'utilisation des protéines par des animaux

(30) Priority: 04.08.1997 NL 1006711
(43) Date of publication of application: 10.02.1999
(73) Proprietor: N.V. Nederlandsche Apparatenfabriek NEDAP, 7141 DC Groenlo (NL)
(72) Inventor: Roosenschoon, Pieter Lieuwe, 7104 BC Winterswijk Meddo (NL)
(74) Representative: Smulders, Theodorus A.H.J.

(56) References cited:
- EP-A- 0 666 475
- DD-A- 228 430
- DE-C- 3 900 119
- US-A- 4 922 855
- US-A- 5 370 885
- PATENT ABSTRACTS OF JAPAN vol. 9, no. 13 (P-328), 19 January 1985 (1985-01-19) & JP 59 160746 A (HITACHI SEISAKUSHO), 11 September 1984 (1984-09-11)

## Description

The invention relates to a system for monitoring and/or controlling protein utilization in animals on a cattle farm, which cattle farm is provided with a milking plant for taking milk from the animals.

The invention also relates to a process for monitoring and/or controlling protein utilization in animals on a cattle farm, which cattle farm is provided with a milking plant for taking milk from the animals.

In cattle farming, it is highly important that the animals (e.g. cows) optimally utilize the protein contained in the ration, because protein is one of the most expensive feed components. The utilization depends, inter alia, on the amount of energy and protein simultaneously present in the animal.

When a ration contains more protein than is necessary, not all the ammonia formed therefrom can be processed in the rumen. The excess ammonia is taken up in the blood and discharged to the liver where the ammonia is converted to urea (=CO(NH₂)₂). This urea is taken up in the blood and largely excreted via the urine. From the blood, a small part of the urea also finds its way to the milk. In a cow producing 25 kg milk, this is about 5 to 8 g (0.2 to 0.3 g/l). The more ammonia in the rumen, the more urea in the blood, and the more urea in the milk.

Excess protein (i.e. also excess nitrogen, because protein includes nitrogen) in the ration leads to a higher urea content in the milk. Accordingly, from the viewpoint of nitrogen utilization, a low urea content is desirable. However, there is also a lower limit. An unduly low urea content indicates an improper energy/urea ratio in the ration or an unduly low protein content of the ration. The animals feed less, produce less milk than they can, and/or the protein content in the milk decreases.

An important point is that it requires energy to process urea. In other words, a high nitrogen balance is energetically unfavorable.

A study has shown that of the feed and animal factors (such as, inter alia, age, lactation stage, race etc.) in particular the uptake of grams of UPB (unstable protein balance) of the total ration significantly influences the urea content. On one farm, substantial differences may occur within a stalling period. These differences particularly occur when changing to another grass silage pit with another UPB. The additional concentrates are then mostly not directly adapted, so that the UPB of the total ration may greatly vary. This becomes manifest in the urea content of the individual animals and of the tank milk.

In the first instance, it is the fresh animals which are important to determine deviations in the energy/protein supply. A second important group is the stale animals. In this group of animals, an unduly low protein yield occurs relatively often in the ration. Sampling of in particular animals from these two groups may be of good help when judging of rations.

Since in many cases concentrate metering computers are used, the observation of a well-balanced energy and protein utilization by the cattle farmer is of essential importance to an optimum automatic application and distribution of the relatively expensive concentrates.

Furthermore, the literature, e.g. the article "Ureumonderzoek in tankmelk voor betere stikstofbenutting" (in translation: "A study on urea in tank milk for better nitrogen utilization") by W.J. Bruins and A.C.G. Beldman (MDM-5, June 1996, Management op Duurzame Melkveebedrijven (in translation: "Management on Permanent Dairy Farms"), a publication by cooperating agricultural institutions), mentions negative effects of high urea contents on fertility. In fact, a high content is associated with conceiving problems. From a level of 30 mg/100 ml already, this becomes evident. Besides, mention is also made of negative effects of high urea contents on the occurrence of lameness (founder) and indigestion.

A study shows that the urea content in milk also gives information about the protein content of the pasture grass and thus indirectly also about the availability of nitrogen to the pasture grass. On the basis of this information, the fertilization of these parcels can be adjusted.

The article "Melkcontrole en managementinfo" (in translation: "Milk inspection and management info") by Dr.Ir. H.J.C.M. van den Bijgaart from the journal Veeteelt (in translation: "Cattle farming") of July 1/2, 1997, clearly indicates the importance of urea determination in milk.

WO 91/00021 also describes a relation between urea content in urine and milk from the animals, on the one hand, and the protein content in the rations of animals, on the other hand, as well as a process for correspondingly adjusting the rations.

EP-A- 0 666 475 discloses a milk checking member which automatically and on-line determines the grade of one or more milk components, such as the albumen and/or fat grades with the aid of sensors during the operation of the milking.

DD-A- 228 430 discloses a flow through electrochemical cell for measuring the urea content in body fluids, such as blood, serum, urine or milk.

The invention is based on the insight that the known process and systems for determining the urea content are not automated. Samples of the milk are taken manually. Consequently, there is only obtained a very limited insight into the protein content in the rations of the animals. Adjusting of the rations to optimize the protein content is therefore rather inaccurate.

It is an object of the system according to the invention to provide a solution to this problem, which system is characterized in that it is provided with a sensor unit which, in use, is arranged to be coupled to the milking plant to automatically measure the urea content of milk contained in the milking plant, and which system is further provided with recording means for automatically recording the measured urea content.

The process according to the invention is characterized in that the urea content of milk contained in the milking plant is automatically measured by means of a sensor unit, and that the automatically measured urea content is recorded.

Since the urea content is now automatically determined and recorded, there can be obtained a very good impression of the protein content in the rations of the animals.

The invention will now be explained in more detail with reference to the accompanying drawings, in which:
Fig. 1 diagrammatically shows the protein digestion in ruminants; and
Fig. 2 shows a possible embodiment of a system according to the invention for carrying out a process according to the invention.

The problem when determining well-balanced rations is that it is necessary to continuously adjust them on the basis of different parameters to be measured. For this purpose, a limited number of cattle farmers presently use, inter alia, the daily milk yield per cow. The consistency of the manure also tells something of the degree of digestion, but, in practice, it can hardly be determined objectively per cow. Besides, the content of fat and protein in the milk is determined every 3 or 4 weeks (milk inspection). The frequency of this determination for regularly adjusting the rations seems too low. Especially in the pasture period with greatly varying feed supplies and relatively long intervals between the milk inspections, much information is lacking. Furthermore, the correlation of fat and protein with the rations is not always very clear.

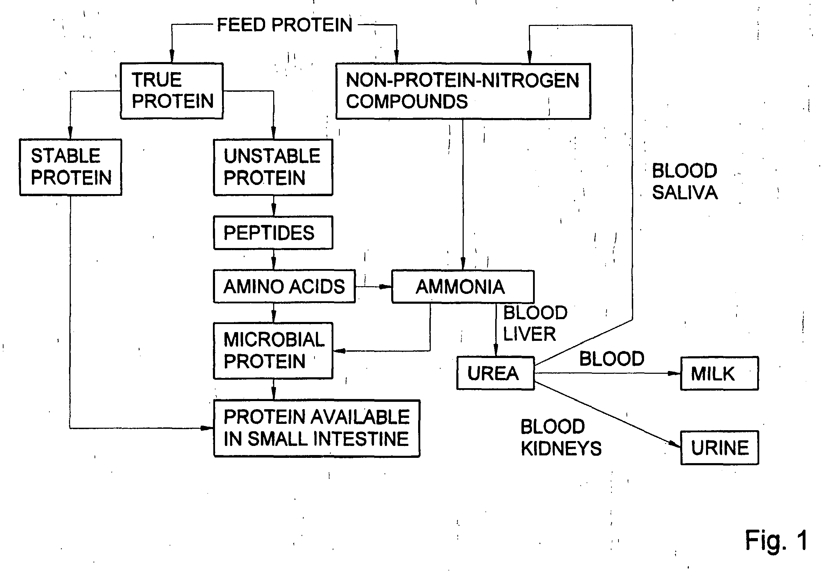

Fig. 1 diagrammatically shows the protein digestion of ruminants. The knowledge of the urea content has an additional effect in this process. When using the urea content as an indicator of nitrogen excess, or protein excess in the rations, continuous observation is very important. At the moment, a cattle farmer can obtain the urea content of individual cows or the bulk tank by taking a sample manually and sending it to a laboratory where it is analyzed. This has the drawback that the cattle farmer receives the result from the laboratory some days later, and not until then can action, if necessary, be taken.

When all the animals receive the same ration, the urea content can also be measured in the tank.

Studies have shown that on the basis of the urea content in the milk the nitrogen losses caused by protein excess in the feed can be properly predicted via the urine. Particularly suitable therefor are samples of groups of animals with the same ration.

Since in the near future the authorities intend to impose strict requirements on the maximum allowable nitrogen losses, a reliable management instrument is required for the cattle farmer to obtain an impression of the nitrogen losses occurring during the production of milk. The measurement of the urea content in milk may be such an instrument.

The object of the present invention is to signalize individual animals or groups of animals in which the urea content in the milk deviates-from normal. This may be a deviation upwards and downwards. The system therefore consists at least of a device mounted in the milk production line (e.g. a long milk tube), or a device capable of sucking up milk from a plant. In the milk sucked up or flowing along, the urea content is determined on-line. This content is compared with an upper or a lower limit. When the actual content exceeds the predetermined limit, this is indicated.

Determining/recording of urea content is possible by means of different advanced techniques, inter alia RAMAN spectroscopy or a conductivity measurement via immobilized enzymes, in which, under the influence of the immobilized enzyme urease, urea is converted to the reaction products ammonium and hydrogen carbonate ions, which can be detected conductometrically on a gold or platinum surface. The latter method is described in the following articles: "Multi-analyte miniature conductance biosensor" by D.C. Cullen, R.S. Sethi and C.R. Lowe in Analytica Chimica Acta 231:33040, 1990 and "Model of an immobilized enzyme conductimetric urea biosensor" by N.F. Sheppard, D.J. Mears and A. Giuseppi-Elie in Biosensors & Bioelectronics 11(10):967-979, 1996.

The latter method is very suitable for use in milk, because the measurement may be carried out on-line during the milking process, without requiring addition of chemical reagents and batch process steps.

By means of the above-mentioned measuring technique, the attention of the cattle farmer is drawn to the indicated animals by giving an audio or visual signal in the milking shed at the animals in question or by showing the indicated animals on a display or a printer.

The data collected by means of such a measuring device are passed to a farm management computer. In this computer, the data obtained are combined with other animal-bound data which are known to be related to the protein utilization. Eligible therefor are the milk production and the concentrate and roughage uptake. The combination of the different date creates a better image of whether or not the protein in the feed is efficiently utilized.

The system according to the invention consists of a urea measuring unit which is combined or coupled by means of a cable with a display housing with a keyboard on which the cattle farmer is notified of, e.g., indicated animals. The measuring unit is provided with two connecting pipes to which are connected the milk supply tube and the milk discharge tube, respectively. It is thereby easy, inter alia, to mount the system in the so-called long milk tube.

Besides, the system may further have the possibility of separating a limited amount of milk in a sample bottle for further analysis in a laboratory. The system can be carried out stand-alone, without coupling with another computer in the farm. In this case, the measuring unit is provided with a central processing unit, a display and a keyboard for inputting threshold values and regularly reading out indications given by the system. It is also possible to couple the system to a so-called farm management computer so as to combine the data concerning the urea content with other data within the system, such as e.g. feed intake, regulation also being possible, and the concentrate and roughage supplies being adjusted regarding quantity and/or type of feed on the basis of the measured urea content in the milk.

On the basis of the urea content in the milk, the nitrogen losses can be predicted via the urine, so that they, inter alia, can be used within the framework of the mineral balance and the manure levy. Furthermore, on the basis of the urea content in the milk, the availability of nitrogen to the pasture grass can be predicted, so that this information can be used to adjust fertilization.

The system can be of mobile design, thus enabling the system to move. Consequently, it is also easy to move a system between different milking positions (animals) or between different farms.

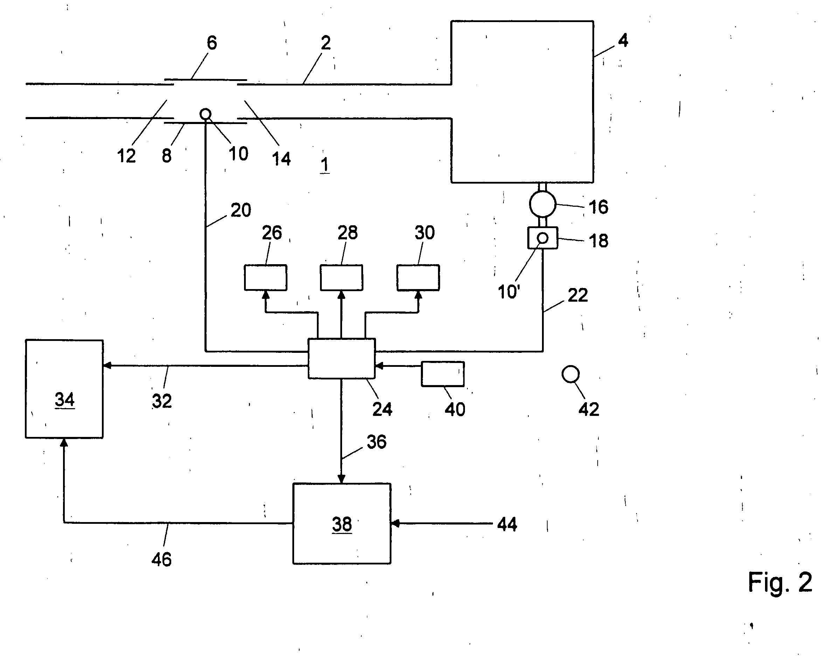

A diagrammatic further elaboration of a system according to the invention for carrying out a process according to the invention is shown in Figure 2.

In Figure 2, numeral 1 denotes a milking plant for taking milk from animals, which is placed in a cattle farm. The milking plant includes at least one milk line 2, which opens into a milk storage container 4. In use, an amount of milk taken from an animal or from a group of animals flows via the milk line 2 to the storage container 4.

The system according to the invention is provided with a first sensor unit 6, which is fitted in the milk line 2 of the milking plant 1. The first sensor unit 6 is provided with a housing 8 having fitted therein a sensor 10 for measuring the urea content of the milk flowing through the milk line 2. The housing 8 has a milk inflow opening 12 and a milk outflow opening 14 for receiving the first sensor unit 6 in the milk line 2. The system is further provided with a pump unit 16 for receiving milk from the milking plant 1. In this case, the pump unit is arranged so as to take, by means of the pump 16, milk from the milk container 4 of the milking plant 1. The device is further provided with a second sensor unit 18 to which the milk taken from the milking plant by means of the pump is supplied for further analysis. The second sensor unit 18 is provided with a sensor 10' of the same type as the sensor 10 discussed before.

The first sensor unit 6 and the second sensor unit 8 are connected to a computer 24 of the system via lines 20 and 22, respectively. Via lines 20 and 22, signals representing urea content, which are generated by the sensors 10 and 10', respectively, are supplied to the computer 24. Connected to the computer 24 are further a printer 26, a display 28 and a loudspeaker 30.

The computer 24 of the system according to the invention is further coupled via line 32 to a feeding plant 34 for automatically giving feed to the animals. Furthermore, the computer 24 is connected via line 36 to a known per se farm computer 38.

The operation of the system according to the invention is as follows.

By means of the first sensor unit, the urea content of milk contained in the milking plant 1, in this example milk flowing through the milk line 2, is measured automatically. By means of the computer 24, the urea content is measured on-line. The computer 24 also records the measured urea content. By means of the pump 16, a sample can likewise be taken from the milk storage container 4 for determining, by means of the second sensor unit 18, the urea content in the sample taken. This measured urea content, too, may be recorded in a storage medium (e.g. a hard disk) of the computer. The computer 24 is arranged to generate a signal when the measured urea content exceeds a predetermined upper or lower limit value. By means of this signal, in this example a visual signal is shown on the display 28 when the measured urea content exceeds the predetermined upper or lower limit value. The display 28 therefore functions as a visual means for giving a visual signal. Quite analogously, the loudspeaker 30 functions as an audio means for giving an audio signal when the measured urea content exceeds the predetermined upper or lower limit value. The computer is further arranged to print information about the measured urea content on the printer 26. When the audio or visual signal is given, a cattle farmer, e.g., may increase the amount of protein in the rations of the animals when the lower limit value is exceeded, or decrease the amount of protein in the rations of the animals when the upper limit value is exceeded.

Furthermore, the system according to the invention may be extended by an identification system 40, 42 of a known per se type. The animals, e.g., are then provided with transponders 42, which contain information about the identity of the animals or the identity of a group of animals. By means of a known per se transmitting and receiving unit 40, this information can be read out and supplied to the computer 24. Thus, the computer 24 can record the urea content belonging to an animal or a group of animals, together with the pertinent identity of the animal or the group of animals. The information recorded in the computer may also be supplied via line 36 to the known per se farm computer 38. The farm computer 38 can process this information, together with other relevant information from the animals, e.g. information about the amount of milk given by the animals, the time when the animals give milk, the amount of feed consumed by the animals etc., to adjust the amount and/or the type of concentrate and/or roughage supply, whether or not automatically or manually. This other relevant information is supplied via line 44 to the farm computer. When this is done automatically, the feeding plant 34 can be correspondingly controlled via a line 46.

Furthermore, the computer 24 may be programmed to generate, on the basis of the measured urea content in the milk, information about nitrogen losses in the urine of the animals. The computer 24 may also be programmed to generate, on the basis of the measured urea content, information about the availability of nitrogen in the pasture grass consumed by the animals. Furthermore, the computer can ensure that the urea content is periodically measured and recorded automatically by means of optionally the first or the second sensor unit, or both.

The sensor 10, 10' may be of any known per se type suitable for determining the urea content. The sensor unit, e.g., may be arranged to determine the urea content in the milk on the basis of the conductivity of ammonium and hydrogen carbonate ions.

The computer 24 and the first sensor unit 6 can be accommodated in a housing. It is also possible to accommodate the computer 24, the pump 16 and the second sensor unit 18 in a housing. The whole system is then of portable design.

## Claims

1. A system for monitoring and/or controlling protein utilization in animals on a cattle farm, which cattle farm is provided with a milking plant for taking milk from the animals, **characterized in that** the system is provided with the milking plant and with a sensor unit which is fitted in a milk line of the milking plant and coupled to the milking plant to automatically and on-line measure the urea content of milk contained in the milking plant during the operation of the milking plant, and which system is further provided with recording means for automatically recording the measured urea content, wherein the system is further provided with a computer for processing signals generated by means of the sensor unit and representing the urea content, and wherein the system generates a signal when the measured urea content exceeds a predetermined upper or lower limit value.

2. A system according to claim 1, **characterized in that** the sensor unit is provided with a housing having included therein a sensor for measuring the urea content, which housing has a milk inflow opening and a milk outflow opening for receiving the first sensor unit in the milk line.

3. A system according to claim 1, **characterized in that** the system is further provided with a pump unit for taking milk from the milking plant and for supplying it to the sensor unit.

4. A system according to anyone of the preceding claims, **characterized in that** the system is further provided with visual means for giving a visual signal when the measured urea content exceeds the predetermined upper or lower limit value.

5. A system according to anyone of the preceding claims, **characterized in that** the system is further provided with audio means for giving an audio signal when the measured urea content exceeds the predetermined upper or lower limit value.

6. A system according to any one of the preceding claims, **characterized in that** the system is further provided with a printer for printing information about the measured urea content.

7. A system according to claim 4 or 6, **characterized in that** the system is arranged such that when the measured urea content exceeds the predetermined lower or upper limit value, the attention of the cattle farmer is drawn thereto by means of an indication list on a display or the printer.

8. A system according to any one of the preceding claims, **characterized in that** the system is provided with a keyboard, a display and a local processing unit and can thus operate stand-alone.

9. A system according to any one of the preceding claims, **characterized in that** the system is further provided with a farm computer coupled with the sensor unit, which farm computer is arranged to process the measured urea content, in combination with other relevant information about the animals.

10. A system according to any one of the preceding claims, **characterized in that** the sensor unit is arranged to determine the urea content in the milk by means of the conductivity of ammonium and hydrogen carbonate ions.

11. A system according to any one of the preceding claims, **characterized in that** the system is of mobile design.

12. A system according to any one of the preceding claims, **characterized in that** the system is further arranged to generate, on the basis of the measured urea content, information for adjusting the amount and/or the type of concentrate and/or roughage supply.

13. A system according to any one of the preceding claims, **characterized in that** the system, independently adjusts the concentrate and roughage supplies regarding amount and/or type of feed, on the basis of the measured urea content in the milk.

14. A system according to any one of the preceding claims, **characterized in that** the system is further arranged to generate, on the basis of the measured urea content in the milk, information about nitrogen losses in urine of the animals.

15. A system according to any one of the preceding claims, **characterized in that** the system is further arranged to generate, on the basis of the measured urea content, information about the availability of nitrogen in the pasture grass consumed by the animals.

16. A system according to any one of the preceding claims, **characterized in that** the system is arranged to measure and record the urea content in the milk per animal or per group of animals:

17. A system according to any one of the preceding claims, **characterized in that** the system is arranged to periodically measure and record the urea content automatically.

18. A process for monitoring and/or controlling protein utilization in animals on a cattle farm, which cattle farm is provided with a milking plant for taking milk from the animals, **characterized in that** the urea content of milk flowing through a milk line of the milking plant of milk contained in the milking plant is measured automatically and on-line during the operation of the milking plant by means of a sensor unit and that the measured urea content is recorded automatically, and that a signal is generated when the measured urea content exceeds a predetermined upper limit value or lower limit value.

19. A process according to claim 18, **characterized in that** the urea content of milk contained in a storage container of the milking plant is measured.

20. A process according to claim 18 or 19, **characterized in that** the signal is an audio and/or visual signal.

21. A process according to any one of the preceding claims 18-20, **characterized in that** information about the measured urea content is printed.

22. A process according to any one of the preceding claims 18-21, **characterized in that** the measured urea content is supplied to a farm computer, which is arranged to process the measured urea content in combination with other relevant information about the animals.

23. A process according to any one of the preceding claims 18-22, **characterized in that**, on the basis of the measured urea content, the amount and/or the type of concentrate and/or roughage supply is adjusted.

24. A process according to any one of the preceding claims 18-23, **characterized in that**, on the basis of the measured urea content in the milk, information about nitrogen losses in urine of the animals is generated.

25. A process according to any one of the preceding claims 18-24, **characterized in that** the urea content is measured and recorded per animal or group of animals.

26. A process according to any one of the preceding claims 18-25, **characterized in that** the urea content is measured periodically.

## Patentansprüche

1. System zum Überwachen und/oder Steuern der Proteinverabreichung an Tiere auf einer Viehfarm, wobei die Viehfarm mit einer Melkanlage zur Entnahme von Milch von den Tieren versehen ist,
**dadurch gekennzeichnet, dass** das System mit der Melkanlage und einer Sensoreinheit versehen ist, die in einer Milchleitung der Melkanlage angeordnet und mit der Melkanlage verbunden ist, um während des Betriebs der Melkanlage den Harnstoffgehalt der in der Melkanlage vorhandenen Milch automatisch und on-line zu messen, und dass das System ferner mit einer Aufzeichnungsvorrichtung zum automatischen Aufzeichnen des gemessenen Harnstoffgehalts versehen ist, wobei das System ferner mit einem Computer zum Verarbeiten von Signalen versehen ist, die mittels der Sensoreinheit erzeugt werden und den Harnstoffgehalt repräsentieren, und wobei das System einen Alarm erzeugt, wenn der gemessene Harnstoffgehalt einen vorbestimmten oberen oder unteren Grenzwert über- bzw. unterschreitet.

2. System nach Anspruch 1, **dadurch gekennzeichnet, dass** die Sensoreinheit mit einem Gehäuse versehen ist, in dem ein Sensor zum Messen des Harnstoffgehalts angeordnet ist, wobei das Gehäuse eine Milcheinflussöffnung und eine Milchausflussöffnung zur Aufnahme der ersten in der Milchleitung angeordneten Sensoreinheit aufweist.

3. System nach Anspruch 1, **dadurch gekennzeichnet, dass** das System ferner mit einer Pumpeinheit versehen ist, um der Melkanlage Milch zu entnehmen und sie der Sensoreinheit zuzuführen.

4. System nach einem der vorherigen Ansprüche, **dadurch gekennzeichnet, dass** das System ferner mit einer optischen Anzeigevorrichtung zur Ausgabe eines optischen Signals versehen ist, wenn der gemessene Harnstoffgehalt den vorbestimmten oberen oder unteren Grenzwert über- bzw. unterschreitet.

5. System nach einem der vorherigen Ansprüche, **dadurch gekennzeichnet, dass** das System ferner mit einer akustischen Anzeigevorrichtung zur Ausgabe eines akustischen Signals versehen ist, wenn der gemessene Harnstoffgehalt den vorbestimmten oberen oder unteren Grenzwert über- bzw. unterschreitet.

6. System nach einem der vorherigen Ansprüche, **dadurch gekennzeichnet, dass** das System ferner mit einem Drucker zum Ausdrucken von Information über den gemessenen Harnstoffgehalt versehen ist.

7. System nach Anspruch 4 oder 6, **dadurch gekennzeichnet, dass** das System derart ausgebildet ist, dass, wenn der gemessene Harnstoffgehalt den vorbestimmten oberen oder unteren Grenzwert über- bzw. unterschreitet, der Viehzüchter darauf mittels einer Angabe-Liste aufmerksam gemacht wird, die über eine Anzeigevorrichtung oder den Drucker verfügbar ist.

8. System nach einem der vorherigen Ansprüche, **dadurch gekennzeichnet, dass** das System mit einer Tastatur, einer Anzeigevorrichtung und einer lokalen Verarbeitungseinheit versehen ist und somit unabhängig arbeiten kann.

9. System nach einem der vorherigen Ansprüche, **dadurch gekennzeichnet, dass** das System ferner mit einem Farm-Computer versehen ist, der mit der Sensoreinheit verbunden ist, wobei der Farm-Computer derart ausgebildet ist, dass er den gemessenen Harnstoffgehalt in Kombination mit weiterer relevanter Information über die Tiere verarbeitet.

10. System nach einem der vorherigen Ansprüche, **dadurch gekennzeichnet, dass** die Sensoreinheit derart ausgebildet ist, dass sie den Harnstoffgehalt in der Milch anhand der Leitfähigkeit von Ammonium- und Hydrogencarbonat-Ionen bestimmt.

11. System nach einem der vorherigen Ansprüche, **dadurch gekennzeichnet, dass** das System mobil ausgebildet ist.

12. System nach einem der vorherigen Ansprüche, **dadurch gekennzeichnet, dass** das System ferner derart ausgebildet ist, dass es auf der Basis des gemessenen Harnstoffgehalts Information zum Einstellen der Menge und/oder des Typs der Konzentrat- und/oder Rauhfutter-Zufuhr erzeugt.

13. System nach einem der vorherigen Ansprüche, **dadurch gekennzeichnet, dass** das System auf der Basis des gemessenen Harnstoffgehalts in der Milch die Konzentrat- und die Rauhfutter-Zufuhr hinsichtlich der zugeführten Menge und/oder des zugeführten Typs unabhängig einstellt.

14. System nach einem der vorherigen Ansprüche, **dadurch gekennzeichnet, dass** das System ferner derart ausgebildet ist, dass es auf der Basis des gemessenen Harnstoffgehalts in der Milch Information zu Stickstoffverlusten im Urin der Tiere erzeugt.

15. System nach einem der vorherigen Ansprüche, **dadurch gekennzeichnet, dass** das System ferner derart ausgebildet ist, dass es auf der Basis des gemessenen Harnstoffgehalts Information zu der Verfügbarkeit von Stickstoff im von den Tieren verzehrten Weidegras erzeugt.

16. System nach einem der vorherigen Ansprüche, **dadurch gekennzeichnet, dass** das System derart ausgebildet ist, dass es den Harnstoffgehalt in der Milch pro Tier oder Gruppe von Tieren misst und aufzeichnet.

17. System nach einem der vorherigen Ansprüche, **dadurch gekennzeichnet, dass** das System derart ausgebildet ist, dass es automatisch den Harnstoffgehalt periodisch misst und aufzeichnet.

18. Verfahren zum Überwachen und/oder Steuern der Proteinverabreichung an Tiere auf einer Viehfarm, wobei die Viehfarm mit einer Melkanlage zur Entnahme von Milch von den Tieren versehen ist,
**dadurch gekennzeichnet, dass** während des Betriebs der Melkanlage der Harnstoffgehalt der Milch, die durch eine Milchleitung der Melkanlage strömt, mittels einer Sensoreinheit automatisch und on-line gemessen wird, und dass ein Signal erzeugt wird, wenn der gemessene Harnstoffgehalt den oberen Grenzwert oder den unteren Grenzwert über- bzw. unterschreitet.

19. Verfahren nach Anspruch 18, **gekennzeichnet durch** das Messen des Harnstoffgehalts von Milch, die in einem Speicherbehälter der Melkanlage enthalten ist.

20. Verfahren nach Anspruch 18 oder 19, **dadurch gekennzeichnet, dass** das Signal ein akustisches und/oder optisches Signal ist.

21. Verfahren nach einem der vorhergehenden Ansprüche 18-20, **dadurch gekennzeichnet, dass** Information zu dem gemessenen Harnstoffgehalt ausgedruckt wird.

22. Verfahren nach einem der vorhergehenden Ansprüche 18-21, **dadurch gekennzeichnet, dass** der gemessene Harnstoffgehalt einem Farm-Computer zugeführt wird, der derart ausgebildet ist, dass er den gemessenen Harnstoffgehalt in Kombination mit weiterer relevanter Information über die Tiere verarbeitet.

23. Verfahren nach einem der vorhergehenden Ansprüche 18-22, **dadurch gekennzeichnet, dass** auf der Basis des gemessenen Harnstoffgehalts die Menge und/oder der Typ der Konzentrat- und/oder Rauhfutter-Zufuhr eingestellt wird.

24. Verfahren nach einem der vorhergehenden Ansprüche 18-23, **dadurch gekennzeichnet, dass** auf der Basis des gemessenen Harnstoffgehalts in der Milch Information zu Stickstoffverlusten im Urin der Tiere erzeigt wird.

25. Verfahren nach einem der vorhergehenden Ansprüche 18-24, **dadurch gekennzeichnet, dass** der Harnstoffgehalt pro Tier oder Gruppe von Tieren gemessen und aufgezeichnet wird.

26. Verfahren nach einem der vorhergehenden Ansprüche 18-25, **dadurch gekennzeichnet, dass** der Harnstoffgehalt periodisch gemessen wird.

## Revendications

1. Système pour contrôler et/ou réguler l'utilisation de protéines chez des animaux d'une exploitation laitière, laquelle exploitation laitière comporte une installation de traite pour traire les animaux, le système étant **caractérisé en ce qu'**il comporte l'installation de traite et un système de détection qui est installé dans un lactoduc de l'installation de traite et est couplé à l'installation de traite pour mesurer automatiquement et en direct le taux d'urée du lait présent dans l'installation de traite pendant le fonctionnement de l'installation de traite, lequel système comporte en outre un moyen d'enregistrement servant à enregistrer automatiquement le taux d'urée mesuré, le système étant en outre pourvu d'un ordinateur pour traiter des signaux générés à l'aide du système de détection et représentant le taux d'urée, et le système produisant un signal lorsque le taux d'urée mesuré dépasse une valeur limite supérieure ou inférieure prédéterminée.

2. Système selon la revendication 1, **caractérisé en ce que** le système de détection est pourvu d'un boîtier dans lequel se trouve un détecteur servant à mesurer le taux d'urée, lequel boîtier comporte une ouverture d'entrée de lait et une ouverture de sortie de lait pour recevoir le premier système de détection dans le lactoduc.

3. Système selon la revendication 1, **caractérisé en ce que** le système comporte en outre un dispositif de pompe pour prélever du lait dans l'installation de traite et le fournir au système de détection.

4. Système selon l'une quelconque des revendications précédentes, **caractérisé en ce que** le système comporte en outre un moyen visuel pour fournir un signal visuel lorsque le taux d'urée mesuré dépasse la valeur limite supérieure ou inférieure prédéterminée.

5. Système selon l'une quelconque des revendications précédentes, **caractérisé en ce que** le système comporte en outre un moyen sonore pour émettre un signal sonore lorsque le taux d'urée mesuré dépasse la valeur limite supérieure ou inférieure prédéterminée.

6. Système selon l'une quelconque des revendications précédentes, le système étant en outre **caractérisé en ce qu'**il comporte une imprimante pour imprimer les informations sur le taux d'urée mesuré.

7. Système selon la revendication 4 ou 6, **caractérisé en ce que** le système est conçu de telle sorte que, lorsque le taux d'urée mesuré dépasse la valeur limite inférieure ou supérieure prédéterminée, l'attention de l'exploitant laitier est attirée sur ce fait au moyen d'une liste d'indications affichée sur un écran ou imprimée par l'imprimante.

8. Système selon l'une quelconque des revendications précédentes, **caractérisé en ce qu'**il comporte un clavier, un écran d'affichage et une unité de traitement local et peut donc fonctionner d'une manière autonome.

9. Système selon l'une quelconque des revendications précédentes, le système étant **caractérisé en ce qu'**il comporte en outre un ordinateur d'exploitation couplé au système de détection, lequel ordinateur d'exploitation est conçu pour traiter le taux d'urée mesuré, en combinaison avec d'autres informations concernant les animaux.

10. Système selon l'une quelconque des revendications précédentes, **caractérisé en ce que** le système de détection est conçu pour déterminer le taux d'urée du lait à l'aide de la conductivité d'ions carbonates d'ammonium et d'hydrogène.

11. Système selon l'une quelconque des revendications précédentes, **caractérisé en ce que** le système est de conception mobile.

12. Système selon l'une quelconque des revendications précédentes, **caractérisé en ce que** le système est en outre conçu pour produire, à partir de la mesure du taux d'urée, des informations servant à ajuster la quantité et/ou le type d'aliments concentrés et/ou de fourrage grossier fournis.

13. Système selon l'une quelconque des revendications précédentes, le système étant **caractérisé en ce qu'**il règle de façon indépendante les apports d'aliments concentrés et de fourrage grossier en ce qui concerne la quantité et/ou le type d'apport, d'après le taux d'urée mesuré dans le lait.

14. Système selon l'une quelconque des revendications précédentes, le système étant **caractérisé en ce qu'**il est en outre conçu pour produire, à partir de la mesure du taux d'urée du lait, des informations sur l'azote éliminé dans l'urine des animaux.

15. Système selon l'une quelconque des revendications précédentes, le système étant en outre **caractérisé en ce qu'**il est conçu pour produire, à partir de la mesure du taux d'urée, des informations sur l'azote disponible dans les graminées de prairies consommées par les animaux.

16. Système selon l'une quelconque des revendications précédentes, le système étant **caractérisé en ce qu'**il est conçu pour mesurer et enregistrer le taux d'urée du lait par animal ou par groupe d'animaux.

17. Système selon l'une quelconque des revendications précédentes, le système étant **caractérisé en ce qu'**il est conçu pour mesurer et enregistrer périodiquement, de manière automatique, le taux d'urée.

18. Procédé pour contrôler et/ou réguler l'utilisation de protéines chez des animaux d'une exploitation laitière, laquelle exploitation laitière est pourvue d'une installation de traite pour traire les animaux, **caractérisé en ce que** le taux d'urée du lait passant dans un lactoduc de l'installation de traite de lait présent dans l'installation de traite est mesuré automatiquement et en direct pendant le fonctionnement de l'installation de traite à l'aide d'un système de détection et **en ce que** le taux d'urée mesuré est enregistré automatiquement, et **en ce qu'**un signal est produit lorsque le taux d'urée mesuré dépasse une valeur limite supérieure ou une valeur limite inférieure prédéterminée.

19. Procédé selon la revendication 18, **caractérisé en ce que** le taux d'urée du lait contenu dans un récipient de stockage de l'installation de traite est mesuré.

20. Procédé selon la revendication 18 ou 19, **caractérisé en ce que** le signal est un signal sonore et/ou visuel.

21. Procédé selon l'une quelconque des revendications précédentes 18 à 20, **caractérisé en ce que** les informations sur le taux d'urée mesuré sont imprimées.

22. Procédé selon l'une quelconque des revendications précédentes 18 à 21, **caractérisé en ce que** le taux d'urée mesuré est fourni à un ordinateur de l'exploitation, qui est conçu pour traiter le taux d'urée mesuré en combinaison avec d'autres informations concernant les animaux.

23. Procédé selon l'une quelconque des revendications précédentes 18 à 22, **caractérisé en ce que**, à partir de la mesure de taux d'urée, la quantité et/ou le type d'aliment concentré et/ou de fourrage grossier fournis sont réglés.

24. Procédé selon l'une quelconque des revendications précédentes 18 à 23, **caractérisé en ce que**, à partir de la mesure du taux d'urée du lait, des informations sur l'azote éliminé dans l'urine des animaux sont produites.

25. Procédé selon l'une quelconque des revendications précédentes 18 à 24, **caractérisé en ce que** le taux d'urée est mesuré et enregistré par animal ou par groupe d'animaux.

26. Procédé selon l'une quelconque des revendications précédentes 18 à 25, **caractérisé en ce que** le taux d'urée est mesuré périodiquement.
